# EUROPEAN PATENT SPECIFICATION

(11) **EP 3 861 929 B1**
(45) Date of publication and mention of the grant of the patent: **25.03.2026**
(21) Application number: 19813065.0
(22) Date of filing: 02.10.2019
(51) Int. Cl.: A61B 5/24, A61B 5/291, A61B 5/316, A61B 5/374, A61B 5/375, G16H 50/30, A61B 5/00

(54) **DEVICE FOR MONITORING AND ASSESSING THE CONDITION OF A PATIENT WITH A NEUROLOGICAL DISORDER IN REAL TIME**
VORRICHTUNG ZUR ÜBERWACHUNG UND BEWERTUNG DES ZUSTANDS EINES PATIENTEN MIT NEUROLOGISCHER STÖRUNG IN ECHTZEIT
DISPOSITIF DE SURVEILLANCE ET D'ÉVALUATION EN TEMPS RÉEL DE L'ÉTAT D'UN PATIENT ATTEINT D'UNE AFFECTION NEUROLOGIQUE

(30) Priority: 03.10.2018 ES 201830954
(43) Date of publication of application: 11.08.2021
(73) Proprietor: MJN Neuroserveis, S.L., 17300 Blanes (ES)
(72) Inventor: BLÁNQUEZ CAUREL, David, 17300 Blanes (ES); RAURICH FÀBREGAS, Xavier, 17300 Blanes (ES); GUTIÉRREZ MORA, Salvador, 17300 Blanes (ES)
(74) Representative: Isern Patentes y Marcas S.L.
(86) International application number: PCT/ES2019/070659
(87) International publication number: WO 2020/070362

(56) References cited:
- US-A1- 2002 103 512
- US-A1- 2004 127 810
- US-A1- 2006 094 974
- US-A1- 2007 213 786
- US-A1- 2008 183 096
- US-A1- 2012 209 101
- US-A1- 2018 000 370

## Description

### OBJECT OF THE INVENTION

The object of the invention application is to register a device configured for carrying out a method for treating neurological disorders of the brain.

More specifically, the invention relates to an apparatus according to claim 1 implemented for detecting and evaluating seizures resulting from neurological conditions, such as for example epileptic seizures, based on measurements of an electroencephalogram (EEG) signal in real time, automatically providing a warning about an imminent seizure, in order to carry out the necessary action to minimise the associated side effects and possible injuries.

### BACKGROUND OF THE INVENTION

Following cerebrovascular accidents, epilepsy is the most common neurological disorder of the brain. It is characterised by recurrent seizures which significantly affect the quality of life of patients. Medicaments are the most common form of treatment, but they have a limited efficacy since they cannot always control seizures, or they cause serious side effects making it impossible to lead a normal life.

A device that is capable of warning about an imminent seizure would allow people with a neurological condition, such as epilepsy, to at least prevent a dangerous situation, such as for example, unexpected falls, blows to the head, injuries, loss of consciousness or lack of balance.

The aid resulting from the measurement of an EEG in the diagnosis and classification of epilepsies is known. However, a correct evaluation thereof is essential to avoid reading more from the measurement, which would involve false diagnoses. Many epileptic patients present a perfectly normal interictal EEG, and occasionally, by using standard derivations, the EEG can be normal even during a complex partial seizure. In contrast, some people present epileptiform paroxysms when they are not experiencing seizures. It is therefore necessary for each seizure prediction method to be customised and optimised for each patient.

Until now several systems have been proposed to improve the quality of life of individuals with epilepsy. For example, patent EP1333753 discloses a device with an

Until now several systems have been proposed to improve the quality of life of individuals with epilepsy. For example, patent EP1333753 discloses a device with an intracranial EEG sensor and a method for automatically predicting and controlling the electrographic onset of a seizure. This system has the drawback that a sensor must be implanted in the head of the patient, with the resulting hospitalisation, surgical intervention, risk and discomfort. Besides that, with the computational capacity available today, it is possible to implement more precise seizure prediction methods than the method disclosed in said document.

Moreover, there are systems with intra-auricular electrodes for measuring EEG signals and configured for warning about hypoglycaemic seizures, such as for example that of patent application WO2011091856, but not for neurological conditions. Document US2012/209101 A1 describes an ear plug adapted for measuring brain wave signals; document US2006/094974 A1 describes a system for monitoring brain waves includes a detection electrode; document US2008/183096 A1 describes a method of monitoring a subject's neurological condition.

The present invention is intended to overcome the drawbacks and limitations of the systems available today.

### DESCRIPTION OF THE INVENTION

The present invention has been developed to provide a device configured to carry out a method for the real-time monitoring and prediction of the state of a patient with a neurological condition, such as epilepsy, which is configured as a novelty in the field of application and solves the aforementioned drawbacks.

The method is based on monitoring the electroencephalogram (EEG) signal of the individual to detect the onset of a seizure of the condition, such as for example of pre-seizure epileptiform activity.

For carrying out the method of the present invention, an auricular device is provided, comprising at least one sensor contacting the inner ear of the patient to measure the EEG and an electronics board, which includes at least one processing unit and one wireless communication unit configured for communicating with a device outside the auricular device. Said auricular device is adapted for carrying out the steps of the method described below, whereby based on the measured EEG signal, a preictal and ictal state in said person is predicted and, depending on said analysed EEG signal, a decision is made as to when an alert is to be provided.

In a preferred embodiment, the sensor is a silver/silver chloride-type electrode integrated in an intra-auricular body. In turn, the auricular device comprises a shell for housing the electronics board and a flexible body attaching which there passes a rod attached to the flexible body, said rod being configured for moving and rotating with respect to the cylindrical body, without being able to be completely removed, while at the same time allowing electrical contact between the sensor and the cables coming from the electronics board.

Regardless of whether or not the device comprises said adapter for adapting the position, the attachment of the intra-auricular body to the cylindrical body or the flexible body, respectively, comprises a ball joint allowing rotation and spherical movement with respect to one another, while at the same time allowing electrical contact between the sensor and the cables coming from the electronics board.

In relation to the method for real-time monitoring of the present invention, said method comprises a first step of measuring the EEG of the patient, performed by said at least one sensor.

Next, the processing unit performs a step of processing the measurements of the EEG, which comprises the usual processes of EEG signal amplification, digitisation and conditioning, said conditioning being, for example, the filtering of possible electrical interferences, atypical values and independent source signals.

The next step is the processing of the conditioned signal, which consists of extracting the values herein called indicative parameters of the state of the patient. This step can be performed by the processing unit itself or, alternatively, it can be performed by an external device, such as the patient's smartphone, prior to the wireless communication between the monitoring device and the external device. These indicative parameters refer to the descriptive parameters measured in the EEG exhibiting a higher correlation with the states of the patient in question. Each person exhibits a different correlation between the measured parameters of his or her EEG and his or her state, therefore said indicative parameters must be selected in a customised manner before implementing the monitoring. The process of selecting the most indicative parameters for each patient is explained below.

It should be pointed out that these indicative parameters can be statistical measurements, energy measurements, complexity measurements and/or connectivity measurements. In this sense, the main parameters could be:
- Band energies;
- Band energy ratio;
- Entropy;
- Mutual information;
- Katz fractal dimension;
- Kolmogorov complexity;
- Means and medians;
- Spectral kurtosis;
- Spectral skewness;
- Cross-correlation;
- Weighted phase lag index;
- Spectral coherence;
- Imaginary coherence; and
- Correlation matrix.

Preferably, in the method according to the invention the value is extracted from at least one of these indicative parameters, where the value can be extracted from another one of the mentioned indicative parameters and/or any other parameter considered indicative for a particular patient based on the process of selecting the most indicative parameters explained below.

For the real-time monitoring process, the EEG timeline is divided into time segments, and a set of values of said indicative parameters is extracted from each segment.

EEG time segmentation allows obtaining finite series of data, thus facilitating detection of the desired events.

This means that the size of the segmentation is preferably defined depending on the event to be detected since, depending on such event, a larger or smaller segmentation time interval will be required, thus preventing the dilution of the event within the series.

Likewise, such segmentation makes it easier for the necessary data processing to also be carried out in portable equipment (preferably, a mobile telephone or the processing unit of the auricular device according to the invention) in an autonomous manner.

Optionally, the EEG timeline is divided into overlapping time segments, i.e., one time segment can share the same section of the EEG with another time segment, whether at the beginning or at the end of the time segment. In other words, the sets of values can share part of their values with other sets of values, such that the measurements of the EEG are extracted and processed in duplicate or the number of times deemed appropriate.

Preferably, the time segments have an overlap window between 20 % and 60 % and, more preferably, around 50 %.

For example, the time segments can consist of 30 seconds (where other segment sizes can be defined) and additionally have an overlap window of 50 % with respect to one another.

The purpose of this overlap of data is to increase the number of samples and allow those events located at the end of one of the time segments overlapping one another to be processed during the following time segment that is part of the overlap.

It should be pointed out that the overlap windows of around 50 % are what allow duplicating the number of windows and samples without unnecessarily repeating data.

From the extracted values, either the external device or the processing unit of the monitoring device performs a step of calculating the risk level of the patient suffering a crisis due to the neurological condition, for one or for several time segments. This calculation is performed by applying Machine Learning, which consists of applying one or several mathematical classification models to each set of extracted values. Among the known mathematical classification models, which are both supervised and unsupervised, are SVM, LSBoost, Random Forest, KNN, Neural Networks, Naive Bayes, K-means, Gaussian process or ANN, although the invention is not limited to these. These models are from the field of artificial intelligence and are based on generating a mathematical model from observations of known classes of a phenomenon in order to predict the class of unknown observations.

In the event of applying more than one mathematical classification model to each set of values, the obtained risk level values are weighted with previously defined weights so as to obtain a single weighted risk level value per time segment.

As can be subsequently verified, these weights can be selected independently for each algorithm and patient according to the best possible solution.

Next, the processing unit performs a step of classifying the segments depending on their risk level, among predefined classifications. These predefined classifications can be, for example, a) preictal and b) non-preictal, where other classifications can be defined, as appropriate. The classification is performed upon comparing the risk level of the segment with a previously defined threshold risk level, whereby the segment is classified as preictal when its risk level is equal to or greater than the threshold level, and classified as non-preictal when the risk level is less than the threshold level.

It should be pointed out that the aforementioned indicative parameters allow better recognition of patterns and assimilation of non-preictal and preictal states.

The state of the patient is classified from the classification of the time segments. In a possible embodiment, the state of the patient is classified as preictal when a single time segment has been classified as preictal. In another embodiment, however, the state of the patient is classified as preictal when there are several time segments that have been classified as preictal, whether consecutive or not, such as for example two consecutive segments, two separated segments in a group of three segments, three or more segments in a group of thirty, etc. By means of this latter preferred embodiment, alterations due to artifacts of the unexpected alerts and signal are avoided.

At the time the state of the patient has been classified as preictal, either the external device gives off a warning signal or else the processing unit transmits an order to the wireless communication unit to send a warning signal to the external device of the patient, as well as to an external device of the person who takes care of said patient, warning about the possibility of suffering a seizure soon.

Optionally, the method comprises a step in which the device informs the patient that his or her state is classified as a state called, for example, a low-risk state, when several time segments comprise a risk level less than the threshold value or a predefined sub-threshold value, which can be, for example, between 1 % and 40 % of what would represent a risk level of 100 %. This is preferably the case, for example, when three consecutive segments occur with a risk level below 40 %. Optionally, the method comprises another step in which the device informs the patient that his or her state is classified as a state called, for example, an intermediate-risk state when one or several time segments comprise a risk level that is between said threshold and sub-threshold values.

It should be pointed out that this avoids generating a sense of uncertainty or causing the patient to become nervous about being notified of an intermediate-risk state more time than necessary and/or without such risk state existing.

After having classified the state of the patient as preictal, given that a pattern differentiating the preictal state from the ictal state of the patient cannot be recognised in the EEG, the method can comprise a step in which a query is transmitted to the patient and/or caregiver whereby the patient must inform through the external device if he or she has suffered a crisis. The query can be transmitted after a sensible time so that, in the event that the patient has suffered a crisis, he or she has been given time to recover, for example after 10 time segments, although preferably after thirty.

Moreover, after having classified the state of the patient as preictal, the method can comprise a step in which the patient is informed that his or her state is classified as an intermediate-risk state when no other time segment is classified as preictal in a group of consecutive segments, said group consisting of at least three consecutive segments, and preferably thirty.

As mentioned, the device can comprise more than one EEG sensor, and in this case, the steps of processing and extracting values can be performed for each measurement of the obtained EEG. The steps of calculating the risk levels and the step of classifying the state of the patient can be performed following different result combinatorial strategies, for example, taking into account a weighting of the risk levels of all time segments coinciding in time. Other strategy examples would be to consider the classifications obtaining the same result for the different sensors as correct or classifying as the preictal state when at least one of the measurements has been classified as preictal.

Another aspect of the present invention, as indicated above, is that the monitoring method comprises a prior method of selecting the variables to be applied, which depend on the biology of each patient, and it serves to customise and optimise the monitoring method.

Said method of selecting variables first includes a process of selecting the mentioned indicative parameters. Given that optimisation of the monitoring method for each patient is sought, those parameters that best differentiate between low-risk and high-risk states, while at the same time eliminating redundant parameters, must be found in each case. This process of selecting indicative parameters comprises a step of measuring a previous EEG, said previous EEG comprising sufficient time segments so that all the possible states of the condition of the patient are recorded. A step of processing these measurements of potential differences obtained by the electrodes is performed, and then a step of obtaining descriptive parameters relative to the EEG, which can be for example, the main frequencies, the band energy, and the statistical values of the time series (such as, inter alia, the average, standard deviation, entropy, connectivity measurements, correlations, coherence, Katz fractal dimension), is performed.

Next, a step of selecting the descriptive parameters exhibiting a higher correlation with the possible states of the patient is performed, and these parameters then become the mentioned indicative parameters. This step is performed by applying one or several of the so-called methods of selecting parameters or variables (not to be confused with the mathematical classification models), such as Neighbourhood Component Analysis, Stepwise Regression, Relief-F and/or Laplacian Score. These methods of selecting variables search for the best subgroup of variables which make the response of the algorithm conform as much as possible to the desired output. For that purpose, they individually analyse each of the variables by observing the reaction of the system when they are included or excluded from the subgroup and by re-classifying samples, comparing the obtained response with the known response. At the end of the analysis, they return a ranking with the significance of each of the variables.

Preferably, to avoid redundant information, a correlation of said selected descriptive parameters is carried out.

Subsequently, the method of selecting variables comprises a process of selecting factors, in which all the possible combinations of factors affecting the calculation of the risk level and the classification of the state of the patient are assessed, finally selecting the combination that is the most consistent with the real states of the patient.

Each of the mathematical classification models has various internal configuration parameters (between 5 and 10) that allow adjusting their behaviour and improving the yield, which may have different values. Moreover, the risk level value obtained by each algorithm can be weighted with that of another algorithm; therefore, the possible combinations of weights must also be assessed. Finally, the value to be assigned to the threshold level whereby a segment is classified as preictal must also be assessed. The factors to be selected, therefore, correspond to:
- the mathematical classification models to be applied in the step of calculating,
- the values of the internal configuration parameters of said models to be applied,
- the weights in the event of applying more than one mathematical classification model, and
- the threshold level to be applied in the step of classifying.

The steps of said process of selecting factors consist of:
- a step of extracting the sets of previous values relative to the indicative parameters of each time segment of the previous EEG,
- establishing a 1^{st} matrix with all the possible combinations for each:
   ∘ possible mathematical classification model applicable to each set of previous values, from a list of known classification algorithms,
   ∘ possible combination of values of the internal configuration parameters of each possible algorithm,
- calculating a 2^{nd} matrix of possible risk levels for each set of previous values by means of each possible algorithm and for each possible combination of the first matrix,
- establishing a 3^{rd} matrix with possible combinations of weights of each possible algorithm,
- calculating a 4^{th} matrix of possible weighted risk levels from:
   ∘ the possible risk levels of the 2^{nd} matrix,
   ∘ the possible combinations of weights of the 3^{rd} matrix,
- establishing a 5^{th} matrix of possible threshold risk levels,
- obtaining a 6^{th} matrix of classification of the hypothetical states of the patient, corresponding to the comparison of each possible weighted risk level of the 4^{th} matrix with each possible threshold risk level of the 5^{th} matrix, wherein said comparisons are designated as a hypothetical alert state (A') when a possible weighted risk level is equal to or exceeds the corresponding possible threshold risk level and as a hypothetical non-alert state (B') when it does not exceed same.
- obtaining a 7^{th} matrix of classification of each combination of factors, corresponding to the comparison of said hypothetical states with the real alert and non-alert states of the patient, the corresponding combination of factors being classified as a hit when the hypothetical and real states coincide, and as a miss when they do not coincide,

Once this last matrix has been obtained, the combination of factors obtaining a higher score in relation to the hits and misses of the seventh matrix is selected from the following hierarchical order of values:
- the ratio of real preictal states that are detected with respect to those that are not detected,
- the ratio of real non-preictal states that are detected with respect to those that are not detected,
- the ratio of hypothetical preictal states that are hits with respect to those that are misses,
- the ratio of hypothetical non-preictal states that are hits with respect to those that are misses,
- the ratio of hits of preictal and non-preictal states balanced with respect to the sample size of each state,
- the ratio of preictal states that are hits and misses with respect to the total samples,
- the ratio of non-preictal states that are hits and misses with respect to the total samples, and
- the amount of previous consecutive segments (TX', TY') classified as non-preictal.

This allows obtaining the best monitoring method for the patient and an optimal customisation thereof.

In order to arrive at the optimal solution, the combinations of factors forming a multidimensional space of good solutions with the best scores can be selected, and the process of selecting factors can be iterated again, modifying the 3^{rd} matrix of possible combinations of weights and the 5^{th} matrix of possible threshold risk levels, with smaller jumps between values of the parameters. The selection of possible factors is performed by means of the grid-search technique, which is a comprehensive combination search technique which allows reaching areas of the multidimensional space where there are various good or acceptable solutions. Moreover, the iteration allows obtaining the most precise combination with the best scores. The number of iterations to search for the best parameters can be between one and fifty.

It should be pointed out that in the end an optimal solution customised for the patient is achieved.

That is, the present invention allows developing a method for the real-time monitoring and evaluation that is trained from the recording of the patient's crises; therefore, it can be customised for each patient.

As mentioned, the device can comprise more than one EEG sensor. For the prior method of selecting variables, their measurements can be combined following different result combinatorial strategies, for example, taking into account a weighting of the risk levels of all time segments coinciding in time, or considering as correct the classifications obtaining the same result for the different sensors.

These and other features and advantages of the present invention will become apparent from the description of a preferred but not exclusive embodiment illustrated by way of non-limiting example in the attached drawings.

### BRIEF DESCRIPTION OF THE DRAWINGS

Figure 1 illustrates an example of a measurement of an electroencephalogram signal with overlapping time segments, where the x-axis represents the time in seconds.
Figure 2 illustrates the flow chart of a possible implementation of the present method for the real-time monitoring and evaluation of the state of a patient, showing on one side a possible method of selecting variables (PS) and on the other a possible implementation of the method for the real-time monitoring (PM). The discontinuous lines refer to the data transferred from one step to another.
Figure 3 illustrates an embodiment of the monitoring device of the present invention.

### BRIEF DESCRIPTION OF A PREFERRED EMBODIMENT

In view of the mentioned drawings and according to the numbering used, the figures show a preferred embodiment of the invention, which comprises the parts and elements indicated and described in detail below.

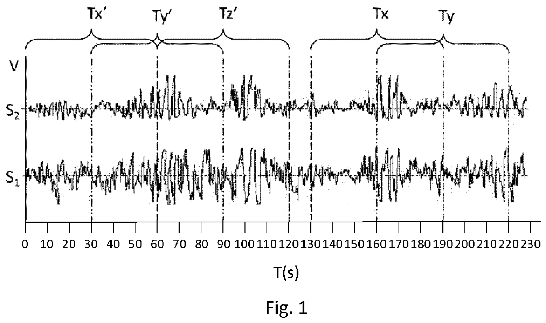

Figure 1 shows an example of a double measurement of the electroencephalogram (EEG) signal of a patient, obtained with a reference electrode (E_{R}) and two intra-auricular sensors (S₁, S₂) detecting brain activity from two different locations in the patient's ear. This figure shows the division of the values of the EEG into time segments (Tx', Ty', Tz', Tx, Ty), on one side two segments (Tx, Ty) relative to the monitoring method (PM), and on the other side three previous time segments (Tx', Ty', Tz') relative to the initial method of selecting the variables (PS), of 60 seconds each and exhibiting an overlap with the next one of 50 %.

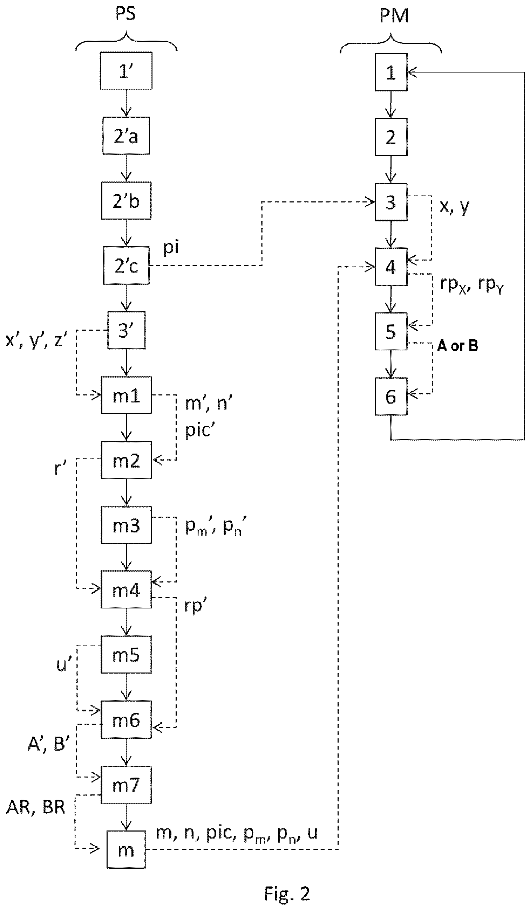

As is shown in Figure 2, the preferred embodiment of the present method of monitoring the state of a patient with a neurological condition comprises an initial method of selecting the variables (PS), depending on the biology of each patient, and it serves to customise and optimise the subsequent monitoring method (PM).

This method of selecting variables (PS) comprises a first process of selecting indicative parameters (pi) including:
- a prior step of measuring (1') the previous EEG of the patient, said EEG comprising sufficient previous time segments (TX', TY') so that all the possible states of the condition of the patient are recorded,
- a prior step of processing (2'a) the measured values of the EEG,
- a step of calculating descriptive parameters (2'b) relative to the previous EEG, and
- a step of applying a method of selecting variables (2'c) to said descriptive parameters for selecting the indicative parameters (pi).

In this preferred embodiment, the applied method of selecting variables is the Neighbourhood Component Analysis.

Moreover, in this embodiment carried out with two EEG sensors (S₁, S₂), all the previous steps are performed in parallel for both obtained measurements. The selected indicative parameters (pi) will be the resultant in any of the two measurements, although they are not repeated in both.

Next, the method of selecting variables (PS) comprises a process of selecting factors (m, n, pic, pm, pn, u) from said indicative parameters (pi), which correspond to:
- the mathematical classification models (m, n) to be applied in the step of calculating (two are indicated in this exemplary embodiment),
- the values of the internal configuration parameters (pic) of said models to be applied,
- the weights (pm, pn) in the event of applying more than one mathematical classification model (m, n), and
- the threshold level (u) to be applied in the step of classifying.

The process of selecting factors comprises a prior step of extracting (3') several sets of values (x', y', z') of the indicative parameters (pi) from each previous time segment (Tx', Ty', Tz'), and then comprises the following steps relative to the search for the multidimensional space with the best combinations of factors:
- establishing a first matrix (m1) with all the possible combinations of values of the internal configuration parameters (pic') of each possible model (m', n') applicable to each set of previous values (x', y', z'),
- calculating a second matrix (m2) of possible risk levels (r') for each set of previous values (x', y') by means of each possible model (m', n') and for each possible combination of the first matrix (m1),
- establishing a third matrix (m3) with possible combinations of weights (pm', pn') of each possible model (m', n'),
- calculating a fourth matrix (m4) of possible weighted risk levels (rp') from:
   ∘ the possible risk levels (r') of the second matrix (m2)
   ∘ the possible combinations of weights (pm', pn') of the third matrix (m3),
- establishing a fifth matrix (m5) of possible threshold risk levels (u'),
- obtaining a sixth matrix (m6) of classification of the hypothetical states of the patient, corresponding to the comparison of each possible weighted risk level (rp') of the fourth matrix (m4) with each possible threshold risk level (u') of the fifth matrix (m5), wherein said comparisons are designated as a hypothetical alert state (A') when a possible weighted risk level (rp') is equal to or exceeds the corresponding possible threshold risk level (U') and as a hypothetical non-alert state (B') when it does not exceed same.
- obtaining a seventh matrix (m7) of classification of each combination of factors, corresponding to the comparison of said hypothetical states (A', B') with real alert state (AR) and real non-alert state (BR) of the patient, the corresponding combination of factors being classified as a hit when the hypothetical and real states coincide, and as a miss when they do not coincide,

Once this last matrix (m7) has been obtained, the combination of factors obtaining a higher score in relation to the hits and misses of the seventh matrix (m7) is selected from the following hierarchical order of values:
- the ratio of real preictal states that are detected with respect to those that are not detected,
- the ratio of real non-preictal states that are detected with respect to those that are not detected,
- the ratio of hypothetical preictal states that are hits with respect to those that are misses,
- the ratio of hypothetical non-preictal states that are hits with respect to those that are misses,
- the ratio of hits of preictal and non-preictal states balanced with respect to the sample size of each state,
- the ratio of preictal states that are hits and misses with respect to the total samples,
- the ratio of non-preictal states that are hits and misses with respect to the total samples, and
- the amount of previous consecutive segments (TX', TY') classified as non-preictal.

Once all the variables of the present monitoring method, i.e., both of the indicative parameters (pi) and of the factors (pic, pm, pn, u), have been selected, it is now possible to apply them to the monitoring method (PM).

As is shown in Figure 2, the preferred embodiment of the monitoring method (PM) initially comprises a step of measuring the EEG (1) of the patient and a step of processing (2) the measured values. Next, for this preferred embodiment, a step of extracting (3) two sets of values (x, y) of indicative parameters (pi) is performed. Each set of values (x, y) corresponds to the values of a time segment (Tx, Ty) of the EEG contained in 60 seconds, the two time segments (Tx, Ty) overlapping by 50 % in this preferred embodiment. Given that the device of this preferred embodiment has two EEG sensors (S₁, S₂), the preceding steps are performed in duplicate, i.e., by each sensor (S₁, S₂). In this case, the two sets of values (x, y) with which the method continues are the mean value of the values previously extracted in duplicate.

The next step corresponds to the step of calculating (4) the risk level of the patient suffering a crisis due to the neurological condition, for each time segment (Tx, Ty) and by means of each mathematical classification model (m, n). By way of example, this calculation can be performed by applying the mathematical classification models (m, n) called SVM and LSBoost to each set of values (x, y). The obtained risk level values for each mathematical classification model (m, n) are weighted with previously defined weights (pm, Pn,) so as to obtain a single weighted risk level value (rpₓ, rp_{y}) for each time segment (Tx, Ty).

Next, there is a step of classifying (5) the state of the patient in each time segment (Tx, Ty), being classified in an alert or preictal state (A) when the weighted risk level (rpₓ, rp_{y}) is greater than the previously defined threshold level (u), and a non-alert or non-preictal state (B) when it is lower.

In this preferred embodiment, the method comprises a step of sending (6) the classification of the state of the patient to a smartphone-type external device. In the event that a time segment (Tx, Ty) has been classified as preictal, the external device transmits a warning signal to the patient and to the person watching over them. In turn, after transmitting the warning signal, a query is transmitted whereby one of the two must confirm if the patient has suffered a crisis.

Once the state of the patient has been classified in a time segment (Tx, Ty), all the steps of the method are repeated again with the following time segments in order to re-classify the state of the patient.

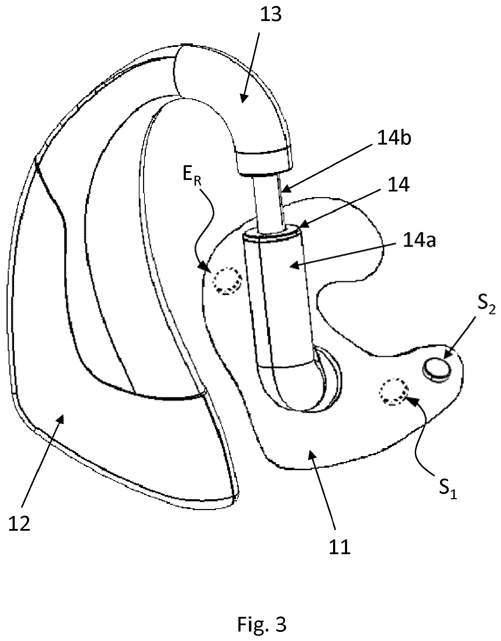

Figure 3 shows an embodiment of the device for the real-time monitoring of the state of a patient with a neurological condition, which is suitable for carrying out the method described above. This auricular device comprises a reference electrode (E_{R}) and two sensors (S₁, S₂), configured for measuring the EEG, all of them being of the silver/silver chloride type and conveniently located in an intra-auricular body (11) so that they can be in direct contact with the right ear of the patient. The figure shows one of the sensors (S₁) and the reference electrode (E_{R}) with dotted lines because they are located on the non-visible face of the intra-auricular body (11). In turn, the device comprises an electronics board (not seen in the figure), which includes a processing unit configured for processing the measurements obtained by the sensors (S₁, S₂) and carrying out the method described above, as well as a wireless communication unit configured for communicating with a device outside the auricular device.

In this preferred embodiment, the device also comprises a shell (12) which houses the electronics board, and a flexible body (13) which attaches the shell (12) to the intra-auricular body (11), said flexible body (13) integrating therein the connection cables between the electronics board and the sensors (S₁, S₂). In turn, this embodiment comprises an adapter for adapting the position (14) between the flexible body (13) and the intra-auricular body (11), provided with a cylindrical body (14a) through which there passes a rod (14b) attached to the flexible body (13), said rod being configured for moving and rotating with respect to the cylindrical body (14a), without being able to be completely removed. At the same time, the adapter (14) allows electrical contact between the sensors (S₁, S₂) and the cables coming from the electronics board. In this case, the attachment between the intra-auricular body (11) and the adapter for adapting the position (14) comprises a ball joint (not seen in the figure) allowing rotation and spherical movement with respect to one another, while at the same time allowing electrical contact between the sensors (S₁, S₂) and the cables coming from the electronics board.

The details, shapes, dimensions and other accessory elements, as well as the materials used in the manufacture of the present monitoring device may be suitably substituted with others that are technically equivalent and do not depart from the essential features of the invention or from the scope defined by the claims included below.

## Claims

1. A device for the real-time monitoring and evaluation of the state of a patient with a neurological condition, comprising:
• at least one sensor (S₁, S₂) located in an intra-auricular body (11) and configured for being in direct contact with the inside of an ear of the patient for measuring the EEG, and
• an electronics board in electrical contact with the sensor (S₁, S₂) including at least one processing unit and one wireless communication unit configured for communicating with a smartphone-type portable device,
wherein the electronics board is configured for carrying out a method for the real-time monitoring and evaluation of the state of a patient with a neurological condition from the indicative parameters (pi) of the state of the patient obtained by means of an EEG comprising:
- a step of measuring the EEG (1) of the patient by means of at least one EEG sensor (S1, S2),
- a step of processing (2) the measured values of the EEG,
- a step of extracting (3) at least one set of values (x, y) of the indicative parameters (pi) of the state of the patient, each set of values (x, y) being extracted from different time segments (Tx, Ty) of the EEG,
- a step of calculating (4) for each time segment (Tx, Ty) the risk level (rpₓ, rp_{y}) of the patient suffering a crisis due to the neurological condition, the risk level (rpₓ, rp_{y}) being calculated by applying at least one mathematical classification model (m, n) to each corresponding set of values (x, y), and
- a step of classifying (5) the state of the patient between at least an alert or preictal state (A) and a non-alert or non-preictal state (B), depending on a predefined threshold level (u), and whereby when the risk level (rpₓ, rp_{y}) in at least one time segment (Tx, Ty) is greater than the threshold level (u), the state of the patient is classified as preictal state (A), and when it is lower it is classified as non-preictal state (B);
wherein this method comprises also a prior process of selecting (PS) the indicative parameters (pi), which comprises:
• a prior step of measuring (1') the previous EEG of the patient, said EEG comprising sufficient previous time segments (Tx', Ty', Tz') so that all the possible states of the condition of the patient are recorded,
• a prior step of processing (2'a) the measured values of the EEG,
• a prior step of calculating descriptive parameters (2'b) relative to the previous EEG, and
• a prior step of applying a method of selecting variables (2'c) to said descriptive parameters;
wherein this method comprises also a prior process of selecting factors, said factors to be selected corresponding to:
• the mathematical classification models (m, n) to be applied in the step of calculating (4),
• the values of internal configuration parameters (pic) of said models (m, n) to be applied,
• the weights (pₘ, pₙ) in the event of applying more than one model (m,n), and
• the threshold level (u) to be applied in the step of classifying (5);
wherein the process of selecting factors comprises the next steps:
• a prior step of extracting (3') sets of previous values (x', y', z') relative to the indicative parameters (pi) of each previous time segment (Tx', Ty', Tz'),
• establishing a first matrix (m1) with all the possible combinations for each:
∘ possible mathematical classification model (m', n') applicable to each set of previous values (x', y', z'), from a list of known models (m', n'),
∘ possible combination of values of the internal configuration parameters (pic') of each possible model (m', n'),
• calculating a second matrix (m2) of possible risk levels (r') for each set of previous values (x', y', z') by means of each possible model (m', n') and for each possible combination of the first matrix (m1),
• establishing a third matrix (m3) with possible combinations of weights (pₘ', pₙ') of each possible model (m', n'),
• calculating a fourth matrix (m4) of possible weighted risk levels (rp') from:
∘ the possible risk levels (r') of the second matrix (m2)
∘ the possible combinations of weights (pₘ', pₙ') of the third matrix (m3),
• establishing a fifth matrix (m5) of possible threshold risk levels (u'),
• obtaining a sixth matrix (m6) of classification of the hypothetical states of the patient, corresponding to the comparison of each possible weighted risk level (rp') of the fourth matrix (m4) with each possible threshold risk level (u') of the fifth matrix (m5), wherein said comparisons are designated as a hypothetical alert state (A') when a possible weighted risk level (rp') is equal to or exceeds the corresponding possible threshold risk level (u') and as a hypothetical non-alert state (B') when it does not exceed same,
• obtaining a seventh matrix (m7) of classification of each combination of factors, corresponding to the comparison of said hypothetical states (A', B') with real alert state (AR) and real non-alert state (BR) of the patient, the corresponding combination of factors being classified as a hit (OK) when the hypothetical and real states coincide, and as a miss (NOK) when they do not coincide,
• selecting the combination of factors obtaining a higher score in relation to a pre-established score depending on the hits and misses of the seventh matrix (m7).

2. The device according to claim 1, which further comprises a shell (12) housing the electronics board, and a flexible body (13) attaching the shell (12) to the intra-auricular body (11), said flexible body integrating therein the connection cables between the electronics board and the sensor (S₁, S₂).

3. The device according to claim 2, wherein the attachment between the intra-auricular body (11) and the flexible body (13) comprises a ball joint allowing rotation and spherical movement with respect to one another, while at the same time allowing electrical contact between the sensor (S1, S2) and the cables coming from the electronics board.

4. The device according to any of the preceding claims, wherein more than one mathematical classification model (m, n) is applied to the sets of values (x, y) of each time segment (Tx, Ty) and the obtained risk level values of each mathematical classification model (m, n) are weighted with previously defined weights (pm, pn) so as to obtain a weighted risk level value (rpₓ, rp_{y}) for each time segment (Tx, Ty).

5. The device according to any of the preceding claims, wherein at least one applied mathematical classification model (m, n) is among those known as SVM, LSBoost, Random Forest, KNN, Neural Networks, Naive Bayes, Gaussian process and ANN.

6. The device according to any of the preceding claims, wherein the sets of values (x, y) are extracted from time segments (Tx, Ty) overlapping one another in an overlap window between 20 % and 60 % of the time.

7. The device according to any of the preceding claims, wherein the method for the real-time monitoring and evaluation of the state of a patient comprises a step wherein a warning signal is transmitted to the patient when 2 or more consecutive time segments (Tx) are classified as preictal (A).

8. The device according to any of the preceding claims, wherein the method for the real-time monitoring and evaluation of the state of a patient comprises a step wherein a warning signal is transmitted to the patient when, in a group of between 3 to 30 consecutive segments, 3 or more time segments (Tx) are classified as preictal (A).

9. The device according to any of the 1 to 6, wherein the method for the real-time monitoring and evaluation of the state of a patient comprises a step wherein a warning signal is transmitted to the patient when a time segment (Tx) is classified as preictal (A).

10. The device according to any of the claims 7 to 9, wherein between 10 to 30 time segments (Tx) after transmitting the warning signal to the patient, a query is transmitted whereby the patient must confirm if he or she has suffered a crisis.

11. The device according to any of the preceding claims, wherein, for the step of measuring the EEG (1) of the method for the real-time monitoring and evaluation of the state of a patient, more than one EEG sensor (S₁, S₂) measures the EEG of the patient, and wherein the steps of processing (2), extracting (3) and calculating (4) by each EEG sensor (S₁, S₂) are duplicated, the step of classifying (5) being performed for all the obtained sets of values (x, y).

## Patentansprüche

1. Vorrichtung zur Überwachung und Beurteilung des Zustands eines Patienten mit einer neurologischen Störung in Echtzeit, umfassend:
• mindestens einen Sensor (S₁, S₂), der sich in einem intraaurikulären Körper (11) befindet und dazu konfiguriert ist, in direktem Kontakt mit dem Inneren eines Ohrs des Patienten zu stehen, um das EEG zu messen, und
• eine Elektronikplatine in elektrischem Kontakt mit dem Sensor (S₁, S₂), die mindestens eine Verarbeitungseinheit und eine drahtlose Kommunikationseinheit enthält, die dazu konfiguriert ist, mit einer tragbaren Vorrichtung vom Typ Smartphone zu kommunizieren,
wobei die Elektronikplatine dazu konfiguriert ist, ein Verfahren zur Überwachung und Beurteilung des Zustands eines Patienten mit einer neurologischen Störung in Echtzeit aus den mittels eines EEGs erhaltenen indikativen Parametern (pi) des Zustands des Patienten durchzuführen, umfassend:
- einen Schritt des Messens des EEG (1) des Patienten mittels mindestens eines EEG-Sensors (S1, S2),
- einen Schritt des Verarbeitens (2) der gemessenen Werte des EEGs,
- einen Schritt des Extrahierens (3) mindestens eines Satzes von Werten (x, y) der indikativen Parameter (pi) des Zustands des Patienten, wobei jeder Satz von Werten (x, y) aus verschiedenen Zeitsegmenten (Tx, Ty) des EEG extrahiert wird,
- einen Schritt des Berechnens (4) für jedes Zeitsegment (Tx, Ty) des Risikoniveaus (rpₓ, rp_{y}) des Patienten, der aufgrund der neurologischen Störung eine Krise erleidet, wobei das Risikoniveau (rpₓ, rp_{y}) durch Anwenden mindestens eines mathematischen Klassifizierungsmodells (m, n) auf jeden entsprechenden Satz von Werten (x, y) berechnet wird, und
- einen Schritt des Klassifizierens (5) des Zustands des Patienten zwischen mindestens einem Alarm- oder Präiktalzustand (A) und einem Nicht-Alarm- oder Nicht-Präiktalzustand (B) in Abhängigkeit von einem vordefinierten Schwellenniveau (u), und wobei, wenn das Risikoniveau (rpₓ, rp_{y}) in mindestens einem Zeitsegment (Tx, Ty) größer als das Schwellenniveau (u) ist, der Zustand des Patienten als Präiktalzustand (A) klassifiziert wird, und wenn er niedriger ist, als Nicht-Präiktalzustand (B) klassifiziert wird;
wobei dieses Verfahren auch einen vorherigen Prozess des Auswählens (PS) der indikativen Parameter (pi) umfasst, der Folgendes umfasst:
• einen vorherigen Schritt des Messens (1') des vorherigen EEG des Patienten, wobei das EEG genügend vorherige Zeitsegmente (Tx', Ty', Tz') umfasst, so dass alle möglichen Zustände der Störung des Patienten aufgezeichnet werden,
• einen vorherigen Schritt des Verarbeitens (2'a) der gemessenen Werte des EEGs,
• einen vorherigen Schritt des Berechnens beschreibender Parameter (2'b) in Bezug auf das vorherige EEG, und
• einen vorherigen Schritt des Anwendens eines Verfahrens zum Auswählen von Variablen (2'c) auf die beschreibenden Parameter;
wobei dieses Verfahren auch einen vorherigen Prozess zum Auswählen von Faktoren umfasst, wobei die auszuwählenden Faktoren Folgendem entsprechen:
• den mathematischen Klassifizierungsmodellen (m, n), die in dem Schritt des Berechnens (4) anzuwenden sind,
• den Werten von internen Konfigurationsparametern (pic) der anzuwendenden Modelle (m, n),
• den Gewichten (pₘ, pₙ) im Falle des Anwendens von mehr als einem Modell (m, n), und
• dem Schwellenniveau (u), das im Schritt des Klassifizierens (5) anzuwenden ist;
wobei der Prozess des Auswählens von Faktoren die nächsten Schritte umfasst:
• einen vorherigen Schritt des Extrahierens (3') von Sätzen vorheriger Werte (x', y', z') in Bezug auf die indikativen Parameter (pi) jedes vorherigen Zeitsegments (Tx', Ty', Tz'),
• Erstellen einer ersten Matrix (m1) mit allen möglichen Kombinationen für jedes bzw. jede:
∘ mögliche mathematische Klassifizierungsmodell (m', n'), das auf jeden Satz vorheriger Werte (x', y', z') anwendbar ist, aus einer Liste bekannter Modelle (m', n'),
∘ mögliche Kombination von Werten der internen Konfigurationsparameter (pic') jedes möglichen Modells (m', n'),
• Berechnen einer zweiten Matrix (m2) möglicher Risikoniveaus (r') für jeden Satz vorheriger Werte (x', y', z') mittels jedes möglichen Modells (m', n') und für jede mögliche Kombination der ersten Matrix (m1),
• Erstellen einer dritten Matrix (m3) mit möglichen Kombinationen von Gewichten (pₘ', pₙ') jedes möglichen Modells (m', n'),
• Berechnen einer vierten Matrix (m4) möglicher gewichteter Risikoniveaus (rp') aus:
∘ den möglichen Risikoniveaus (r') der zweiten Matrix (m2)
∘ den möglichen Kombinationen von Gewichten (pₘ', pₙ') der dritten Matrix (m3),
• Erstellen einer fünften Matrix (m5) möglicher Schwellenrisikoniveaus (u'),
• Erhalten einer sechsten Matrix (m6) der Klassifizierung der hypothetischen Zustände des Patienten, die dem Vergleich jedes möglichen gewichteten Risikoniveaus (rp') der vierten Matrix (m4) mit jedem möglichen Schwellenrisikoniveau (u') der fünften Matrix (m5) entspricht, wobei die Vergleiche als hypothetischer Alarmzustand (A') bezeichnet werden, wenn ein mögliches gewichtetes Risikoniveau (rp') gleich dem entsprechenden möglichen Schwellenrisikoniveau (u') ist oder dieses überschreitet, und als hypothetischer Nicht-Alarmzustand (B'), wenn es dieses nicht überschreitet,
• Erhalten einer siebten Matrix (m7) der Klassifizierung jeder Kombination von Faktoren, die dem Vergleich der hypothetischen Zustände (A', B') mit dem realen Alarmzustand (AR) und dem realen Nicht-Alarmzustand (BR) des Patienten entspricht, wobei die entsprechende Kombination von Faktoren als ein Treffer (OK) klassifiziert wird, wenn die hypothetischen und realen Zustände übereinstimmen, und als ein Fehlschlag (NOK), wenn sie nicht übereinstimmen,
• Auswählen der Kombination von Faktoren, die einen höheren Zahlenwert im Verhältnis zu einem vorher festgelegten Zahlenwert erhalten, in Abhängigkeit von den Treffern und Fehlschlägen der siebten Matrix (m7).

2. Vorrichtung nach Anspruch 1, die ferner ein Gehäuse (12), das die Elektronikplatine aufnimmt, und einen flexiblen Körper (13) umfasst, der das Gehäuse (12) an dem intraaurikulären Körper (11) befestigt, wobei der flexible Körper darin die Verbindungskabel zwischen der Elektronikplatine und dem Sensor (S₁, S₂) integriert.

3. Vorrichtung nach Anspruch 2, wobei die Befestigung zwischen dem intraaurikulären Körper (11) und dem flexiblen Körper (13) ein Kugelgelenk umfasst, das eine Drehung und eine sphärische Bewegung zueinander ermöglicht, während es gleichzeitig einen elektrischen Kontakt zwischen dem Sensor (S1, S2) und den von der elektronischen Vorrichtung kommenden Kabeln ermöglicht.

4. Vorrichtung nach einem der vorhergehenden Ansprüche, wobei mehr als ein mathematisches Klassifizierungsmodell (m, n) auf die Sätze von Werten (x, y) jedes Zeitsegments (Tx, Ty) angewendet wird und die erhaltenen Risikoniveauwerte jedes mathematischen Klassifizierungsmodells (m, n) mit zuvor definierten Gewichten (pm, pn) gewichtet werden, um einen gewichteten Risikoniveauwert (rpₓ, rp_{y}) für jedes Zeitsegment (Tx, Ty) zu erhalten.

5. Vorrichtung nach einem der vorhergehenden Ansprüche, wobei mindestens ein angewandtes mathematisches Klassifizierungsmodell (m, n) zu denen gehört, die als SVM, LSBoost, Random Forest, KNN, Neuronale Netzwerke, Naive Bayes, Gauß-Prozess und ANN bekannt sind.

6. Vorrichtung nach einem der vorhergehenden Ansprüche, wobei die Sätze von Werten (x, y) aus Zeitsegmenten (Tx, Ty) extrahiert werden, die sich in einem Überlappungsfenster zwischen 20 % und 60 % der Zeit überlappen.

7. Vorrichtung nach einem der vorhergehenden Ansprüche, wobei das Verfahren zur Überwachung und Beurteilung des Zustands eines Patienten in Echtzeit einen Schritt umfasst, bei dem ein Warnsignal an den Patienten übermittelt wird, wenn 2 oder mehr aufeinanderfolgende Zeitsegmente (Tx) als präiktal (A) klassifiziert werden.

8. Vorrichtung nach einem der vorhergehenden Ansprüche, wobei das Verfahren zur Überwachung und Beurteilung des Zustands eines Patienten in Echtzeit einen Schritt umfasst, bei dem ein Warnsignal an den Patienten übermittelt wird, wenn in einer Gruppe von 3 bis 30 aufeinanderfolgenden Segmenten 3 oder mehr Zeitsegmente (Tx) als präiktal (A) klassifiziert werden.

9. Vorrichtung nach einem der 1 bis 6, wobei das Verfahren zur Überwachung und Beurteilung des Zustands eines Patienten in Echtzeit einen Schritt umfasst, bei dem ein Warnsignal an den Patienten übermittelt wird, wenn ein Zeitsegment (Tx) als präiktal (A) klassifiziert wird.

10. Vorrichtung nach einem der Ansprüche 7 bis 9, wobei zwischen 10 und 30 Zeitsegmenten (Tx) nach dem Übermitteln des Warnsignals an den Patienten eine Abfrage übermittelt wird, bei der der Patient zu bestätigen hat, falls er oder sie eine Krise erlitten hat.

11. Vorrichtung nach einem der vorhergehenden Ansprüche, wobei für den Schritt des Messens des EEG (1) des Verfahrens zur Überwachung und Beurteilung des Zustands eines Patienten in Echtzeit mehr als ein EEG-Sensor (S₁, S₂) das EEG des Patienten misst und wobei die Schritte des Verarbeitens (2), des Extrahierens (3) und des Berechnens (4) durch jeden EEG-Sensor (S₁, S₂) dupliziert sind, wobei der Schritt des Klassifizierens (5) für alle erhaltenen Sätze von Werten (x, y) durchgeführt wird.

## Revendications

1. Dispositif pour la surveillance et l'évaluation en temps réel de l'état d'un patient souffrant d'une affection neurologique, comprenant :
• au moins un capteur (S₁, S₂) situé dans un corps intra-auriculaire (11) et conçu pour être en contact direct avec l'intérieur d'une oreille du patient pour mesurer l'EEG, et
• une carte d'électronique en contact électrique avec le capteur (S₁, S₂) comportant au moins une unité de traitement et une unité de communication sans fil configurée pour communiquer avec un appareil portable de type téléphone intelligent,
dans lequel la carte d'électronique est configurée pour mettre en œuvre un procédé pour la surveillance et l'évaluation en temps réel de l'état d'un patient souffrant d'une affection neurologique à partir des paramètres indicatifs (pi) de l'état du patient obtenus au moyen d'un EEG, comprenant :
- une étape de mesure de l'EEG (1) du patient au moyen d'au moins un capteur d'EEG (S1, S2),
- une étape de traitement (2) des valeurs mesurées de l'EEG,
- une étape d'extraction (3) d'au moins un ensemble de valeurs (x, y) des paramètres indicatifs (pi) de l'état du patient, chaque ensemble de valeurs (x, y) étant extrait de différents segments temporels (Tx, Ty) de l'EEG,
- une étape de calcul (4) pour chaque segment temporel (Tx, Ty) du niveau de risque (rpₓ, rp_{y}) que le patient souffre d'une crise due à l'affection neurologique, le niveau de risque (rpₓ, rp_{y}) étant calculé en appliquant au moins un modèle de classification mathématique (m, n) à chaque ensemble correspondant de valeurs (x, y), et
- une étape de classification (5) de l'état du patient entre au moins un état d'alerte ou préictal (A) et un état non alerte ou non préictal (B), en fonction d'un niveau de seuil prédéfini (u), et moyennant quoi lorsque le niveau de risque (rpₓ, rp_{y}) dans au moins un segment temporel (Tx, Ty) est supérieur au niveau de seuil (u), l'état du patient est classifié comme état préictal (A), et lorsqu'il est inférieur, il est classifié comme état non préictal (B) ;
dans lequel ce procédé comprend également un processus préalable de sélection (PS) des paramètres indicatifs (pi), qui comprend :
• une étape préalable de mesure (1') de l'EEG antérieur du patient, ledit EEG comprenant un nombre suffisant de segments temporels antérieurs (Tx', Ty', Tz') de sorte que tous les états possibles de l'affection du patient soient enregistrés,
• une étape préalable de traitement (2'a) des valeurs mesurées de l'EEG,
• une étape préalable de calcul de paramètres descriptifs (2'b) par rapport à l'EEG antérieur, et
• une étape préalable d'application d'un procédé de sélection de variables (2'c) auxdits paramètres descriptifs ;
dans lequel ce procédé comprend également un processus préalable de sélection de facteurs, lesdits facteurs à sélectionner correspondant :
• aux modèles de classification mathématique (m, n) à appliquer dans l'étape de calcul (4),
• aux valeurs de paramètres de configuration internes (pic) desdits modèles (m, n) à appliquer,
• aux poids (pₘ, pₙ) en cas d'application de plus d'un modèle (m,n), et
• au niveau de seuil (u) à appliquer dans l'étape de classification (5) ;
dans lequel le processus de sélection de facteurs comprend les étapes suivantes :
• une étape préalable d'extraction (3') d'ensembles de valeurs antérieures (x', y', z') par rapport aux paramètres indicatifs (pi) de chaque segment temporel antérieur (Tx', Ty', Tz'),
• établissement d'une première matrice (m1) avec toutes les combinaisons possibles pour chaque :
∘ modèle de classification mathématique (m', n') possible applicable à chaque ensemble de valeurs antérieures (x', y', z'), à partir d'une liste de modèles (m', n') connus,
∘ combinaison possible de valeurs des paramètres de configuration internes (pic') de chaque modèle (m', n') possible,
• calcul d'une deuxième matrice (m2) de niveaux de risque possibles (r') pour chaque ensemble de valeurs antérieures (x', y', z') au moyen de chaque modèle (m', n') possible et pour chaque combinaison possible de la première matrice (m1),
• établissement d'une troisième matrice (m3) avec des combinaisons possibles de poids (pₘ', pₙ') de chaque modèle (m', n') possible,
• calcul d'une quatrième matrice (m4) de niveaux de risque pondérés possibles (rp') à partir de :
∘ les niveaux de risque possibles (r') de la deuxième matrice (m2)
∘ les combinaisons possibles de poids (pₘ', pₙ') de la troisième matrice (m3),
• établissement d'une cinquième matrice (m5) de niveaux de risque seuils possibles (u'),
• obtention d'une sixième matrice (m6) de classification des états hypothétiques du patient, correspondant à la comparaison de chaque niveau de risque pondéré possible (rp') de la quatrième matrice (m4) avec chaque niveau de risque seuil possible (u') de la cinquième matrice (m5), dans lequel lesdites comparaisons sont désignées comme un état d'alerte hypothétique (A') lorsqu'un niveau de risque pondéré possible (rp') est égal au ou dépasse le niveau de risque seuil possible (u') correspondant et comme un état de non-alerte hypothétique (B') lorsqu'il ne le dépasse pas,
• obtention d'une septième matrice (m7) de classification de chaque combinaison de facteurs, correspondant à la comparaison desdits états hypothétiques (A', B') avec l'état d'alerte réel (AR) et l'état de non-alerte réel (BR) du patient, la combinaison correspondante de facteurs étant classifiée comme un succès (OK) lorsque les états hypothétique et réel coïncident, et comme un échec (NOK) lorsqu'ils ne coïncident pas,
• sélection de la combinaison de facteurs obtenant un score plus élevé par rapport à un score préétabli en fonction des succès et des échecs de la septième matrice (m7).

2. Dispositif selon la revendication 1, qui comprend en outre une coque (12) abritant la carte d'électronique, et un corps flexible (13) fixant la coque (12) au corps intra-auriculaire (11), ledit corps flexible intégrant à l'intérieur les câbles de connexion entre la carte d'électronique et le capteur (S₁, S₂).

3. Dispositif selon la revendication 2, dans lequel la fixation entre le corps intra-auriculaire (11) et le corps flexible (13) comprend une articulation sphérique permettant la rotation et le mouvement sphérique l'un par rapport à l'autre, tout en permettant en même temps un contact électrique entre le capteur (S1, S2) et les câbles provenant de la carte d'électronique.

4. Dispositif selon l'une quelconque des revendications précédentes, dans lequel plus d'un modèle de classification mathématique (m, n) est appliqué aux ensembles de valeurs (x, y) de chaque segment temporel (Tx, Ty) et les valeurs de niveau de risque obtenues de chaque modèle de classification mathématique (m, n) sont pondérés avec des poids antérieurement définis (pm, pn) de manière à obtenir une valeur de niveau de risque pondérée (rpₓ, rp_{y}) pour chaque segment temporel (Tx, Ty).

5. Dispositif selon l'une quelconque des revendications précédentes, dans lequel au moins un modèle de classification mathématique (m, n) appliqué est parmi ceux connus comme SVM, LSBoost, Forêt aléatoire, KNN, Réseaux neuronaux, Bayésien naïf, processus gaussien et ANN.

6. Dispositif selon l'une quelconque des revendications précédentes, dans lequel les ensembles de valeurs (x, y) sont extraits des segments temporels (Tx, Ty) se chevauchant l'un l'autre dans une fenêtre de chevauchement comprise entre 20 % et 60 % du temps.

7. Dispositif selon l'une quelconque des revendications précédentes, dans lequel le procédé pour la surveillance et l'évaluation en temps réel de l'état d'un patient comprend une étape au cours de laquelle un signal d'avertissement est transmis au patient lorsqu'au moins 2 segments temporels (Tx) consécutifs sont classifiés comme préictaux (A).

8. Dispositif selon l'une quelconque des revendications précédentes, dans lequel le procédé pour la surveillance et l'évaluation en temps réel de l'état d'un patient comprend une étape au cours de laquelle un signal d'avertissement est transmis au patient lorsque, dans un groupe d'entre 3 à 30 segments consécutifs, 3 segments temporels (Tx) ou plus sont classifiés comme préictaux (A).

9. Dispositif selon l'une quelconque des 1 à 6, dans lequel le procédé pour la surveillance et l'évaluation en temps réel de l'état d'un patient comprend une étape au cours de laquelle un signal d'avertissement est transmis au patient lorsqu'un segment temporel (Tx) est classifié comme préictal (A).

10. Dispositif selon l'une quelconque des revendications 7 à 9, dans lequel entre 10 et 30 segments temporels (Tx) après la transmission du signal d'avertissement au patient, une requête est transmise moyennant quoi le patient doit confirmer s'il a souffert d'une crise.

11. Dispositif selon l'une quelconque des revendications précédentes, dans lequel, pour l'étape de mesure de l'EEG (1) du procédé pour la surveillance et l'évaluation en temps réel de l'état d'un patient, plus d'un capteur d'EEG (S₁, S₂) mesure l'EEG du patient, et dans lequel les étapes du traitement (2), d'extraction (3) et de calcul (4) par chaque capteur d'EEG (S₁, S₂) sont dupliquées, l'étape de classification (5) étant réalisée pour tous les ensembles obtenus de valeurs (x, y).
